# EUROPÄISCHE PATENTSCHRIFT

(11) **EP 0 626 020 B1**
(45) Veröffentlichungstag und Bekanntmachung des Hinweises auf die Patenterteilung: **06.08.1997**
(21) Anmeldenummer: 93903154.8
(22) Anmeldetag: 04.02.1993
(51) Int. Cl.: D04B 1/26, A61F 13/08

(54) **GESTRICK**
KNITTED FABRIC
TRICOT

(30) Priorität: 11.02.1992 DE 9201860 U
(43) Veröffentlichungstag der Anmeldung: 30.11.1994
(73) Patentinhaber: WEIHERMÜLLER & VOIGTMANN GMBH & CO KG, D-95449 Bayreuth (DE)
(72) Erfinder: WEIHERMÜLLER, Stefan, D-8580 Bayreuth 24 (DE)
(74) Vertreter: Voigt, Günter, Dipl.-Ing.
(86) Internationale Anmeldenummer: DE9300107
(87) Internationale Veröffentlichungsnummer: WO9316222

(56) Entgegenhaltungen:
- EP-A- 0 045 372
- EP-A- 0 105 773
- DE-A- 3 832 798
- DE-U- 8 606 402
- DE-U- 8 903 749
- DE-U- 9 201 860
- FR-A- 2 435 546
- US-A- 3 250 095
- US-A- 3 717 150
- PATENT ABSTRACTS OF JAPAN vol. 015, no. 440 (C-0883)11. November 1991

## Beschreibung

Die Erfindung bezieht sich auf ein Gestrick gemäß Oberbegriff des Anspruchs 1.

Ein solches Gestrick ist grundsätzlich bereits aus der US-A 3 250 095 und aus der EP-A 0 045 372 bekannt. Bei diesen bekannten Gestricken weist die innere hautnahe Schicht eine geringere Feuchtigkeitsaufnahmefähigkeit als die weiter außen liegende Schicht aus einem Faden aus vielkapillarigem Material auf. Diese Gestricke weisen nicht das Merkmal auf, daß die Anzahl der Kapillare in der weiter außen liegenden Schicht gleich oder größer als die Zahl der Garnstärke in dtex ist, wobei eine Schicht mit flottliegenden Fäden als weitere, oben erwähnte hautnahe innere Schicht fungiert.

Aus dem Artikel "Des tricots fonctionnels pour le sport" in der Zeitschrift "L'INDUSTRIE TEXTILE" , No 1218, Fevrier 1991, ist es weiter bekannt, die Materialien mit der Garnstärke laut kennzeichnendem Teil aus Polyester und Polyamid 66 für die Herstellung von Sportkleidung zu verwenden und dabei die feinsten Filamente in die äußere Oberfläche zu verlegen, um so die Feuchtigkeitsabtransport von innen nach außen zu fördern. Es handelt sich jedoch um ein Verarbeitungsprodukt aus flachen Stoffbahnen, die dann für das zu fertigende Produkt zugeschnitten und anschließend zusammengenäht werden

Die Besonderheiten einer Verarbeitung eines nahtlos maschinengestrickten Strumpfes mittels Rundstrickmaschinen werden hier nicht berücksichtigt. Tatsächlich galt die Verarbeitung solcher Materialien auf Rechts-Links-Kleinrundstrickmaschinen auch bisher als nicht möglich oder zumindest als nicht praktikabel.

Aus dem Artikel "Microfasern - Modewelle oder Standard von morgen ?" (Melliand Textilberichte 12/1991, Seiten 971 ff., insbesondere Seite 977, Absatz 3 ist darüber hinaus auch die Rolle von Micro-Filamentgarnen als Funktionsschicht für den Feuchtetransport in Zwei-Schicht-Strickwaren bekannt.

Auf das Grundgestrick aufgebrachte Frotteegewebe - wie sie in der Praxis auch schon zur Anwendung kamen - weisen den Nachteil auf, daß sie eine von dem Grundgestrick abweichende Elastizität besitzen und damit die Elastizität des Strumpfes in dem von ihnen belegten Bereich beeinflussen. Darüberhinaus kommt es wegen des unterschiedlichen Dehnungsverhaltens des Grundgestrickes einerseits und der aufgesetzten Frotteegewebe andererseits langfristig häufig zu Ablösungen des Frotteegewebes vom Grundgestrick.

Der Erfindung liegt die Aufgabe zugrunde, ein Gestrick für Kompressionsstrümpfe, Stützstrümpfe oder Damenfeinstrümpfe bzw. entsprechende Hosen zu schaffen, das bei Aufrechterhaltung der vollen Elastizität einen verbesserten Transport der Feuchtigkeit des Körpers von der Innenseite des Gestricks zu dessen Außenseite ermöglicht.

Die Lösung dieser Aufgabe erfolgt mit den Merkmalen des Anspruchs 1.

Vorteilhafte Weiterbildungen ergeben sich aus den Unteransprüchen.

Die Erfindung wird nachfolgend unter Bezugnahme auf die Zeichnungsfiguren beispielsweise erläutert. Es zeigen:
- Fig. 1: eine schematische Darstellung eines Gestrickes, bei dem ein Zusatzfaden über mehrere Maschen des Gestrickes nicht abgebunden ist, sowie
- Fig. 2: eine schematische Darstellung des Aufbaus eines dreischichtigen Gestricks.

Kompressionsstrümpfe, Stützstrümpfe oder Damenfeinstrümpfe (bzw. die entsprechenden Hosen) werden mittels Rundstrickmaschinen hergestellt..

Für den vorliegenden Verwendungszweck wird die Ware auf einer Ein-Zylinder-Rundstrickmaschine gefertigt.

Hier ist es besonders schwierig ein zwei- oder mehrflächiges Gestrick zu erzeugen, erheblich schwieriger als auf einer Rechts/Rechts-Strickmaschine, wie sie beispielsweise für Oberbekleidung Verwendung findet.

Es hat sich als besonders vorteilhaft erwiesen, mehrere Schichten unterschiedlicher Feuchtigkeitsaufnahmefähigkeit direkt miteinander zu verbinden bzw. zu verstricken.

Es werden daher zwei oder mehr Stricksysteme verwendet. Das erste Stricksystem wird zweifädig gearbeitet, wobei ein Faden mit separatem Fadenführer glatt gestrickt wird. Das hierbei verwendete Material liegt auf der Außenseite des Gestricks. Ein zweiter Faden wird mit separatem Fadenführer flottliegend als Bogen oder Schlaufe auf der Innenseite des Gestricks eingebunden. Dies geschieht über eine spezielle Nadelauswahl, die über eine Mustereinrichtung gesteuert wird.

So können beispielsweise jeweils eine erste Nadel den Faden verstricken und eine zweite Nadel den Faden flott, also ungebunden, verarbeiten; es können aber auch jeweils mehr Nadeln den Faden verstricken und über mehr Nadeln den Faden flott verarbeiten.

Bei einem weiteren Stricksystem wird ein zweiflächiger Charakter eines Rechts/Links-Gestricks - ähnlich einem Rechts/Rechts-Gestrick - erreicht, um einen Feuchtigkeitstransport vom Faden auf der Innnenseite zu dem auf der Außenseite zu gewährleisten. Durch einfaches Plattieren (Hinzufügen eines Fadens) kann diese Eigenschaft nicht erreicht werden.

Von erheblicher Bedeutung ist auch die Struktur der verwendeten Fäden. Der Faden auf der innenliegenden (der hautnahen) Seite des Gestricks soll weniger Filamente (Fibrillen) aufweisen - z.B.: dtex 78 f 23 - als der oder die weiter außen liegenden Fäden.

In der oder den weiter außen liegenden Schichten wird ein Material verwendet - z,B.: dtex 78 f 68, dtex 85 f 95 - das die Feuchtigkeit der weiter innen liegenden Schicht aufnehmen, somit weiter nach außen transportieren kann. Aus der ganz außen liegenden Schicht kann die Feuchtigkeit dann an die Umgebung abgegeben werden.

Hierzu muß die jeweils weiter außenliegende Schicht aus einem vielkapillarigen Material aufgebaut sein. das eine größere Filamentenzahl aufweist als die weiter innen liegende Schicht. Damit wird von Schicht zu Schicht eine von innen nach außen steigende Aufnahmefähigkeit für die zu transportierende Feuchtigkeit geschaffen und so der Transport eben dieser von innen nach außen erreicht.

Stärke und Material der einzelnen Schichten richten sich nach dem jeweiligen Verwendungszweck. Die Fäden können glatt gestrickt sein oder flott liegen.

Im letzten Fall ergeben sich im ungedehnten Zustand der Ware leichte Bögen oder Schlaufen. Dies ist erwünscht und wird erreicht durch eine spezielle Nadelauswahl während des Strickvorganges. Der flottliegende Faden 14 wird nicht von jeder Nadel erfaßt - wie das übrige Gestrick 15 - sondern nur von jeder zweiten, dritten oder n-ten Nadel.

Die Nadelauswahl erfolgt durch eine spezielle Mustereinrichtung an der Maschine. Dies kann über sogenannte Schnabelstößer, Musterschieber und Zwischenschieber geschehen.

Der flottliegende Faden 14 kann verschiedene Titer, d.h. Stärken, aufweisen. Er muß über einen besonders dafür zugerichteten Fadenführer den Nadeln zugeführt werden, wobei Höhe und Position und damit der Einlaufwinkel des Fadens zur Nadelauswahl entscheidend sind. Auch die Bremsung und damit die Spannung des Fadens hat einen wesentlichen Einfluß auf die Schlingenbildung und wird über eine besonders konstruierte Fadenbremseinrichtung erzeugt.

Das Grundgestrick kann aus einem oder mehreren elastomeren Fäden oder aus Fäden mit elastomeren Bestandteilen bestehen. Auch der flottliegende Faden 14 kann möglicherweise elastomere Bestandteile aufweisen. Es muß jedoch gewährleistet sein, daß die gewünschten Eigenschaften dadurch nicht (wesentlich) beeinträchtigt werden.

Sofern ein Strumpfteil vorhanden ist, kann ein farbiger Kennstreifen der Orientierung über die vorgesehene Lage der Ferse dienen. Die durch den farbigen Kennstreifen gegebene Markierung verläuft von der Innenseite des Fußes über die Ferse zur Außenseite des Fußes und reicht so vorzugsweise über die Hälfte des Umfangs des Strumpfes in diesem Bereich.

Für das Einstricken des Streifens werden keine Pendelungen ausgeführt, so daß der übliche Rundstrickvorgang nicht unterbrochen wird. Wenn für die Herstellung des schmalen Streifens im Flächenabschnitt der Ferse ein andersartiger, gegebenenfalls auch andersfarbiger Faden verwendet wird, ist der Fersenbereich des Strumpfes (der auch Teil einer Strumpfhose sein kann) für das richtige Anlegen eindeutig festgelegt. Die Lage der Ferse ist damit optisch ohne weiteres erkennbar.

Eine weitere Verbesserung der feuchtigkeitsabführenden Wirkung kann durch ein dreischichtiges Gestrick erreicht werden. Es werden dann zwei Fäden miteinander plattiert und ein dritter - innen- oder außenliegend - flottgelegt. Es können auch zwei Fäden innen und außen flott liegen.

Bei allen Stricksystemen muß gewährleistet sein. daß ein Feuchtigkeitstransport von innen nach außen erfolgt. Nachfolgend werden zwei vorteilhaft verwendbare Stricksysteme näher beschrieben.

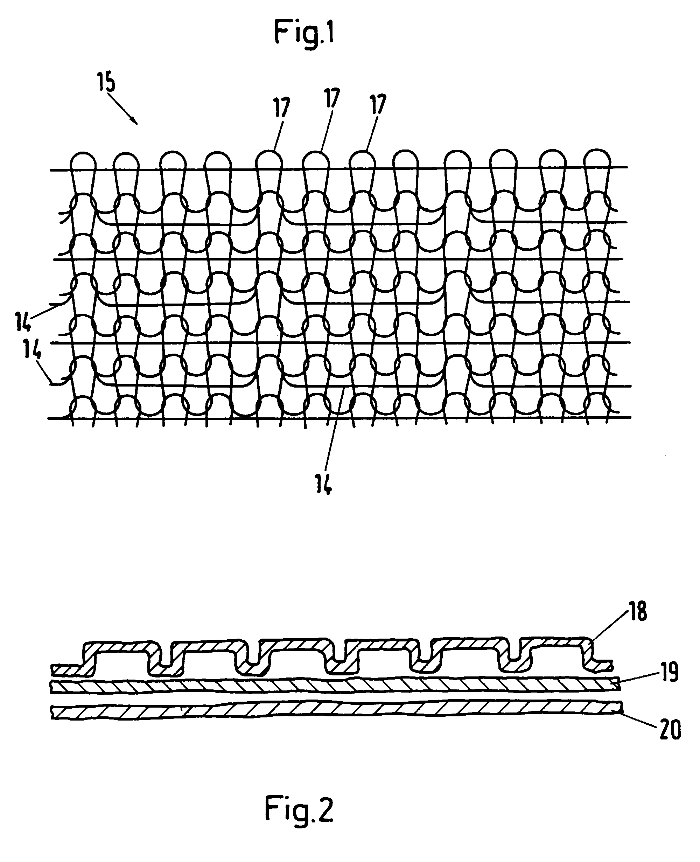

Bei einem dreischichtigen System gemäß Fig. 2 wird der Faden der ersten Schicht 18 mit speziellem Fadenführer an die durch Mustereinrichtung, Schieber, Zwischenschieber oder Schnabelstöße ausgewählte Nadel heran und dieser zugeführt. Das zum Einsatz kommende Material kann aus herkömmlichen Materialien (Helanca oder elastischen Fäden) oder aus allen Chemie- oder Naturfasern bestehen, wobei die Fadenstärke (Titer) auf das jeweilige Einsatzgebiet (Strumpf, Hose, Zwickel) abgestimmt werden muß. Das Material der Schicht 18 sollte keine Feuchtigkeit speichern.

Die zweite - in der Warenmitte liegende - Schicht 19 hilft den zu erzielenden Effekt (trockene Seite innen feuchte Seite außen) zu verstärken. Deshalb ist der in dieser Schicht zu verarbeitende Faden in Charakter und Stärke (Titer) auf die benachbarten Schichten abgestimmt und findet sowohl für den Feuchtigkeitstransport als auch für die Feuchtigkeitsabgabe an die weiter außen liegende Schicht Verwendung. Die Zuführung des Fadens zur Nadel geschieht mit separatem Fadenführer und einer Bindung "R/L glatt".

In der dritten Schicht 20 ist dann der Plattierfaden oder der flottliegende Faden allein für die Feuchtigkeitsabgabe an die Umgebung zuständig. Das zu verwendende Garnmaterial ist eine Microfaser, bei der die Fibrillenzahl (Kapillare) gleich oder größer ist als die Garnstärke (Titer). Der Plattierfaden oder der flottliegende Faden werden mit separatem Fadenführer der Nadel derart zugeführt. daß der Faden der weiter innen liegenden zweiten Schicht 19 abgedeckt (plattiert) wird. Die Garnstärke der Microfaser richtet sich nach den jeweiligen Einsatzgebieten des fertigen Produkts (Strumpf. Strumpfhose oder Zwickel). Die Bindung ist in diesem Fall "R/L plattiert" oder flottiert. Die Fadenführung erfolgt über einen speziellen Fadenführer.

Um ein feuchtigkeitsabführendes (klimatisierendes) Leibteil zu fertigen, müssen auch die einzelnen Schichten (Wareninnenseite. Warenaußenseite) in Bezug auf die Fadenstärke aufeinander abgestimmt sein. Dies gilt grundsätzlich sowohl für zweischichtige als auch für dreischichtige Gestricke. Bei diesem Abstimmungsprozeß hat auch die Maschengröße (Maschendichte) eine wichtige Funktion, da das Hosenteil - insbesondere bei Kompressionsstrumpfhosen - für die verschiedensten Körperweiten und -längen verwendbar sein muß.

Bei einem anderen Stricksystem kann ebenfalls ein zweioder dreischichtiges Gestrick gewählt werden. Wie beim zuvor beschriebenen ersten Stricksystem sind Material. Fadenführung und Nadelauswahl in der ersten Schicht aufeinander abzustimmen. In der zweiten Schicht wird das gleiche Garnmaterial wie in der ersten Schicht verwendet. Der Strickfaden dieser Schicht erhält die Bindung "R/L glatt".

Um eine Kompressionswirkung bei einem mehrschichtigen Gestrick zu erzeugen, kann zusätzlich noch ein Schußfaden verarbeitet werden, der hauptsächlich aus Polyurethan mit Chemiefaser- oder Naturfaser-Umspinnung oder nacktem Lycra bestehen kann. Die Bindung erfolgt 1:1, d.h. an einer Nadel wird der Faden eingelegt, bei der nächsten Nadel hinterlegt. Die Nadelauswahl kann auch im Verhältnis 1:2, 2:2 oder in einer anderen Variante festgelegt werden.

Besondere Bedeutung kommt der Fertigung des Zwickels im Hosenteil zu. Dieser soll grundsätzlich in allen Belangen mit dem Hosenteil (Leibteil) identisch sein. In bestimmten Fällen kann der Zwickel aber auch in besonderer Weise im Hinblick auf die feuchtigkeitsabführende Wirkung ausgebildet sein. D.h. es kann unter bestimmten Umständen allein der Zwickel eine solche feuchtigkeitsabführende Wirkung haben oder aber dieser Teil der Hose ist in besonderem Maße auf diese gewünschte Wirkung ausgelegt.

Von Bedeutung für die Feuchtigkeitsleitfähigkeit sind auch die Färbevorgänge. Diese müssen so gestaltet werden, daß der gewünschte Feuchtigkeitstransport im Gestrick von innen nach außen voll erhalten bleibt und nicht durch chemische Zusätze oder Hilfsmittel beeinträchtigt wird.

## Patentansprüche

1. Mehrschichtiges hochelastisches Gestrick für medizinische Strümpfe, Strumpfhosen, Zwickel, Damenfeinstrümpfe oder ähnliches, wobei die hautnahe innere Schicht (18) eine geringere Feuchtigkeitsaufnahmefähigkeit als die weiter außen liegende Schicht (19. 20) aufweist und die weiter außen liegende Schicht (19, 20) aus einem Faden aus vielkapillarigem Material besteht, dadurch gekennzeichnet, daß die Ware aus elastischen oder elastomeren Garnen rundgestrickt ist, deren Anzahl der Einzelfilamente in der weiter außen liegenden Schicht (19, 20) in etwa gleich oder größer als die Zahl der Garnstärke in dtex ist, wobei eine Schicht mit flottliegenden Fäden (14) als weitere, oben erwähnte hautnahe innere Schicht fungiert.

2. Gestrick nach Anspruch 1, dadurch gekennzeichnet, daß die Anzahl der Kapillare gleich oder größer als die Zahl der Garnstärke (dtex) ist.

3. Gestrick nach Anspruch 1 oder 2, dadurch gekennzeichnet, daß drei oder mehr Schichten vorhanden sind.

4. Gestrick nach einem der Ansprüche 1 bis 3, dadurch gekennzeichnet, daß die innere Schicht mit einem Faden aus handelsüblichem Polyamid gestrickt ist.

5. Gestrick nach einem der Ansprüche 1 bis 4, dadurch gekennzeichnet, daß der Faden für die innere Schicht eine Feinheit im Bereich dtex 78 f 23 aufweist.

6. Gestrick nach einem der Ansprüche 1 bis 5, dadurch gekennzeichnet, daß die weiter außen liegenden Schichten aus einem Faden mit einer Feinheit im Bereich dtex 78 f 78 gestrickt sind.

7. Gestrick nach einem der Ansprüche 1 bis 5, dadurch gekennzeichnet, daß die weiter außen liegenden Schichten aus einem Faden mit einer Feinheit im Bereich dtex 85 f 92 gestrickt sind.

## Claims

1. Multilayer, highly elastic knitware for medical stockings, tights, gussets, fine ladies' stockings or the like, wherein the inner layer (18) close to the skin displays a lower moisture absorption capacity than the layer (19, 20) lying further outwards and the layer (19, 20) lying further outwards consists of a thread of multicapillary material, characterised thereby, that the article is circularly knitted of elastic or elastomeric yarns, the number of the single filaments of which in the layer (19, 20) lying further outwards is about equal to or greater than the number of the yarn thickness in dtex, wherein one layer with floating threads (14) functions as further, aforementioned inner layer close to the skin.

2. Knitware according to claim 1, characterised thereby, that the number of the capillaries is equal to or greater than the number of the yarn thickness (dtex).

3. Knitware according to claim 1 or 2, characterised thereby, that three or more layers are present.

4. Knitware according to one of the claims 1 to 3, characterised thereby, that the inner layer is knitted with a thread of commercially usual polyamide.

5. Knitware according to one of the claims 1 to 4, characterised thereby, that the thread for the inner layer displays a fineness in the region of dtex 78 f 23.

6. Knitware according to one of the claims 1 to 5, characterised thereby, that the layers lying further outwards are knitted of a thread with a fineness in the region of dtex 78 f 78.

7. Knitware according to one of the claims 1 to 5, characterised thereby, that the layers lying further outwards are knitted of a thread with a fineness in the region of dtex 85 f 92.

## Revendications

1. Tricot de haute élasticité à plusieurs couches pour des bas médicaux, des collants, des pièces en pointes, des bas fins pour dames ou des produits similaires, où la couche intérieure (18) qui est au contact de la peau présente une moindre capacité d'absorption de l'humidité que la couche extérieure (19), (20) qui est tricotée à partir d'un fil multifilaments caractérisé en ce que le produit en fils élastiques ou d'élastomère est tricoté sur un métier circulaire, le nombre de filaments individuels de la couche extérieure (19), (20) étant environ égal ou plus grand que le titre de finesse du fil en dtex, et, par une autre couche, intérieure, au contact de la peau, telle qu'indiquée ci-dessus, qui est constituée de fils flottants (14).

2. Tricot selon la revendication 1 caractérisé en ce que le nombre de filaments individuels est égal ou supérieur au titre de finesse du fil en dtex.

3. Tricot selon les revendications 1 ou 2 caractérisé par trois ou plusieurs couches.

4. Tricot selon l'une des revendications 1 à 3 caractérisé en ce que la couche intérieure est tricotée avec un fil de polyamide usuel.

5. Tricot selon l'une des revendications 1 à 4 caractérisé en ce que le fil utilisé pour la couche intérieure a une finesse comprise dans la zone dtex 78 f 23.

6. Tricot selon l'une des revendications 1 à 5 caractérisé en ce que les couches extérieures sont tricotées avec un fil présentant une finesse comprise dans la zone dtex 78 f 78.

7. Tricot selon l'une des revendications 1 à 5 caractérisé en ce que les couches extérieures sont tricotées avec un fil présentant une finesse comprise dans la zone dtex 85 f 92.
